# EUROPEAN PATENT SPECIFICATION

(11) **EP 2 612 630 B1**
(45) Date of publication and mention of the grant of the patent: **04.03.2015**
(21) Application number: 11821846.0
(22) Date of filing: 31.08.2011
(51) Int. Cl.: A61F 13/02, A61K 9/70, A61L 15/58

(54) **ADHESIVE SKIN PATCH SHEET**
KLEBEFOLIE FÜR EIN HAUTPFLASTER
FEUILLE ADHÉSIVE POUR TIMBRE TRANSDERMIQUE

(30) Priority: 31.08.2010 WO PCT/JP2010/064824
(43) Date of publication of application: 10.07.2013
(73) Proprietor: Nichiban Co. Ltd., Tokyo 112-8663 (JP)
(72) Inventor: OKADA Masaki, Tokyo 112-8663 (JP); TAKEZAKI Akihito, Tokyo 112-8663 (JP); KAJIWARA Takahiro, Tokyo 112-8663 (JP)
(74) Representative: Jones, Helen M.M.
(86) International application number: PCT/JP2011/069714
(87) International publication number: WO 2012/029831

(56) References cited:
- WO-A1-96/21410
- JP-A- 5 038 348
- JP-A- 10 118 174
- JP-A- H10 511 881
- JP-A- 2001 114 672
- JP-A- 2003 033 389
- US-A- 3 038 597
- US-A- 5 437 622

## Description

### TECHNICAL FIELD

The present invention relates to a skin patch sheet including an adhesive patch (refers to both the case where a pharmacologically active substance is incorporated in an adhesive layer and the case where a pharmacologically active substance is not incorporated). More particularly, the present invention relates to a skin patch sheet by which an adhesive patch having a thin backing can be adhered to a surface to be attached of skin or the like with good operability by using one hand, without having the adhesive brought into contact with the finger, and which produces fewer waste materials when patch attachment has been finished. More preferably, the present invention relates to a skin patch sheet including an adhesive patch that is used to prevent direct scratching of an affected area of skin by being attached to the skin surface, or to a skin patch sheet including an adhesive patch for cosmetic applications that is used for a lift-up effect by being attached to the face.

### BACKGROUND ART

Adhesive patches that are used by being attached to skin surfaces are usually provided with an adhesive layer on one surface of a flexible backing such as a plastic film or a fabric, and are utilized in various applications such as first-aid adhesive tapes, bandages, dressings, skin surface fixing sheets, and transdermally absorbed medicated adhesive patches.

Conventional adhesive patches are such that the thickness of the backing is about 20 to 200 µm, and the backing has rigidity sufficient to backing the adhesive per se and to enable attachment to a target site of the skin or the like.

On the other hand, in thin adhesive patches such as dressing materials, flexible transparent films having a thickness of approximately 20 µm and having low rigidity may be used as the backing. This is because, even after an instrument or the like is fixed to skin, detachment of a tube or an injection needle, skin rashes at the fixing site, and the like can be made visible through the transparent film.

Furthermore, when an adhesive patch is attached to face, neck, arms, legs, and the like, if a transparent film having a thickness of approximately 20 µm is used as the backing, and the adhesive patch is made hardly noticeable, effects in cosmetic applications or effects of enhancing the QOL of patients may be obtained, so that the added value of the adhesive patch can be increased.

If the thickness of the backing is 20 µm or less, the ability to maintain the shape of the backing itself is decreased, and at the time of attaching, the adhesive patch may have creases, or the backing may be broken. Thus, it has been a conventional practice that in the case of using a thin backing having a thickness of 20 µm or less in an adhesive patch, a carrier sheet is detachably attached to the backing.

JP 7-132139 A (Patent Literature 1) discloses a guide film-attached adhesive patch having a layer configuration in which a thermoplastic polyester polyurethane film having a thickness of 5 to 50 µm is used as a backing, an acrylic adhesive layer obtained by copolymerizing pyrrolidone and having a thickness of 1 to 20 µm is provided on one surface of the backing, and a guide film formed from a polyethylene film, a polyethylene terephthalate film or the like is detachably laminated on the other surface of the backing. WO 2009/41122 A (Patent Literature 2) discloses, as an adhesive patch which is suitable for wrinkles or fine surface unevenness of skin and makes the site of attachment unnoticeable, an adhesive patch in which an adhesive layer having a thickness of 1 to 15 µm is provided on one surface of a base material layer formed from an elastomer film having a thickness of 1 to 10 µm, while a carrier layer is disposed on the base material layer on the reverse side of the adhesive layer.

In Patent Literatures 1 and 2, it is a technically common item to form a carrier sheet on the backing for the purpose of enhancing the handleability of an adhesive patch having a backing which is not firm. However, during the period from the point after peeling an adhesive patch from a release material or a separator layer to the point of attaching the adhesive patch to the surface to be attached of skin or the like, fingers may touch the adhesive surface, so that there has been room for an improvement in terms of handleability.

JP 2003-277254 A (Patent Literature 3) discloses an adhesive patch agent for external use, of which a therapeutic skin patch can be easily peeled from a release paper and can be easily attached to an affected site. JP 2009-28137 A (Patent Literature 4) discloses a medical skin patch set, in which the skin patch can be easily detached from a release liner, the skin patch can be attached without touching the adhesive surface, and a required number of skin patches can be conveniently taken out when needed.

In Patent Literatures 3 and 4, it is a technical common item that the skin patch can be attached to the skin without the finger touching the adhesive surface, by gripping a pickup piece or pull tab and detaching the skin patch from the release liner. However, these skin patches do not assume lamination of a carrier sheet on the backing. Even if a detachable carrier sheet could be provisionally attached on the backing of the skin patch, in that case, an operation of detaching the release liner from the skin patch, subsequently attaching the skin patch to the skin, and then detaching the carrier sheet, is required. Therefore, plural waste materials such as the release liner and the carrier sheet are produced, and the peeling operation or waste disposal operation become complicated.

JP 5-38348 A (Patent Literature 5) discloses an adhesive sheet having a backing member, a backing, an adhesive layer, and a separator in this order, in which the portion of the adhesive sheet from the separator to the backing can be partitioned by at least one slit line. Patent Literature 5 discloses that as the procedure of attaching the adhesive sheet to human skin, the adhesive patch is attached to the skin, and then the separator remaining in the periphery, the adhesive layer and backing that are adjacent thereto are removed by peeling, and the remaining backing member is separately detached to accomplish attachment. The operation of waste disposal includes several steps, that is, at least two or more times, and the operation of waste disposal is still complicated.

WO96/21419 (Patent Literature 6) discloses an adhesive sheet having a carrier sheet, an adhesive patch and a release liner laminated in this order, wherein a slit line is provided. In use, the backing sheet is removed from the laminate by gripping the backing sheet tab and pulling the backing sheet tab away from the carrier layer to detach the backing sheet from the carrier sheet; and the holding portion is then removed from the positioned carrier sheet.

### CITATION LIST

### PATENT LITERATURE

Patent Literature 1: JP 7-132139 A
Patent Literature 2: WO 2009/41122 A
Patent Literature 3: JP 2003-277254 A
Patent Literature 4: JP 2009-28137 A
Patent Literature 5: JP 5-38348 A
Patent Literature 6: WO96/21419

### SUMMARY OF INVENTION

### TECHNICAL PROBLEM

The present invention was achieved in view of such circumstances, and it is an object of the present invention to provide a skin patch sheet by which, when an adhesive patch having a thin backing is attached to an object to be attached such as the skin surface, the adhesive patch can be easily attached without contaminating the adhesive surface by the finger or the like, and the operation of waste disposal is completed in one stage until attachment is finished.

### SOLUTION TO PROBLEM

The inventors of the present invention conducted thorough investigations on the structure of a skin patch sheet including a carrier sheet, an adhesive patch including a thin backing, and a release liner, and as a result, the inventors found that the object described above may be achieved by providing a particular slit which extends from the side of the release liner that is larger in area than the adhesive patch including a backing, penetrating into the adhesive patch, up to the middle of the thickness direction of the carrier sheet.

According to the present invention, there is provided a skin patch sheet comprising a carrier sheet, an adhesive patch and a release liner laminated in this order, wherein
(a) the adhesive patch comprises two layers of a backing and an adhesive layer having the same shape,
(b) the carrier sheet is adjacent to the backing of the adhesive patch,
(c) the skin patch sheet comprises a grip section and a body section,
(d) in the body section, the area of the release liner is larger than the area of the adhesive patch,
(e) the boundary between the grip section and the body section is a slit line formed by a slit, and
(f) the slit cuts into the skin patch sheet from the release liner side, penetrating into the adhesive patch, to a position at which at least half or more of the thickness of the carrier sheet on the adhesive patch is left uncut. In an embodiment the slit extends from the side of the release liner, penetrating into the adhesive patch, up to a depth in the range of greater than or equal to 20% and less than 50% of the thickness on the adhesive patch side of the carrier sheet.

Furthermore, according to embodiments of the present invention, the following skin patch sheet is provided.
(1) The skin patch sheet, in which plural sheets of the adhesive patches, each having a carrier sheet laminated thereon, are arranged in parallel on the release liner.
(2) The skin patch sheet, in which plural sheets of the adhesive patches, each having a carrier sheet laminated thereon, are arranged in parallel on the release liner, and the outer periphery of the grip section is formed by being connected to the slit line.
(3) The skin patch sheet, in which in the grip section, the area of the release liner is larger than the area of the adhesive patch.
(4) The skin patch sheet, in which the grip section includes only a carrier sheet.
(5) The skin patch sheet, in which the slit of (f) does not penetrate into the release liner.
(6) The skin patch sheet, in which the thickness of the backing is 1 to 10 µm.
(7) The skin patch sheet, in which the backing is an elastomer film.
(8) The skin patch sheet, in which the backing is a polyurethane elastomer film.
(9) The skin patch sheet, in which the carrier sheet is a polyester film, a polyolefin film or a plastic laminated paper, and the release liner is a polyester film, a polyolefin film or a plastic laminated paper, all of which are release-treated.
(10) The skin patch sheet, in which the adhesive patch has an approximately rectangular shape where the ratio of lengths of the shorter edge and the longer edge is 1:2 to 1:9, and the slit line is formed in a direction parallel to the shorter edge at a distance in the range of 1/8 to 1/3 of the length of the longer edge of the adhesive patch.
(11) The skin patch sheet, in which the vicinities of vertexes of the adhesive patch having an approximately rectangular shape are cut out to an approximately arc shape.
(12) The skin patch sheet, in which the carrier sheet in the grip section forms an angle of 1 to 90° with respect to the extended direction of the surface of the body section, with the slit line serving as the fulcrum.
(13) The skin patch sheet, in which the peeling force required to detach the carrier sheet from the backing is larger than the peeling force required to detach the release liner from the adhesive layer.
(14) The skin patch sheet, in which a pharmacologically active substance is included in the adhesive layer.
(15) The skin patch sheet, in which the adhesive patch is used by being attached to the face.
(16) The skin patch sheet, in which the adhesive patch is used by being attached to the face for a lift-up effect.
(17) The skin patch sheet used to prevent direct scratching of the skin.

Furthermore, use of the skin patch sheet, in which the adhesive patch is attached to the face for a lift-up effect, or use of the skin patch sheet, in which the adhesive patch is attached to the skin surface in order to prevent direct scratching of the skin is provided.

Further, a method for attaching the adhesive patch of the skin patch sheet to an object to be attached is provided. The method includes: holding the grip section; detaching the adhesive patch of the body section having a carrier sheet laminated thereon, from the release liner; attaching the adhesive patch having a carrier sheet laminated thereon, to an object to be attached; and subsequently finishing the operation of attachment by detaching the grip section and the carrier sheet.

### ADVANTAGEOUS EFFECTS OF INVENTION

Since the skin patch sheet of the present invention is a skin patch sheet in which a carrier sheet, an adhesive patch, and a release liner are laminated in this order, in which:
(a) the adhesive patch includes two layers of a backing and an adhesive layer having the same shape;
(b) the carrier sheet is adjacent to the backing of the adhesive patch;
(c) the skin patch sheet includes a grip section and a body section;
(d) in the body section, the area of the release liner is larger than the area of the adhesive patch;
(e) the boundary between the grip section and the body section is a slit line formed by a slit; and
(f) the slit extends from the side of the release liner, penetrating into the adhesive patch, up to a depth in the range of greater than or equal to 20% and less than 50% of the thickness on the adhesive patch side of the carrier sheet, an effect that the attachment operability of the adhesive patch to the object to be attached such as skin is excellent, and the adhesive patch can be neatly attached without creases, is provided.

Furthermore, the skin patch sheet of the present invention provides an effect that when the adhesive patch is attached to an object to be attached, the surface of the adhesive layer is not contaminated with the finger, detachment of the carrier sheet can be conveniently carried out as compared with conventional skin patch sheets, and the operation of waste disposal until the attachment is finished may be reduced.

The skin patch sheet of the present invention can be utilized as various skin patch sheets for medical applications, cosmetic applications, industrial applications, stationery applications, domestic applications, and the like by utilizing these features.

### BRIEF DESCRIPTION OF DRAWINGS

Fig. 1 is a plan view of a specific example of the skin patch sheet according to the present invention. A slit X forms a slit line which serves as a boundary between a grip section a and a body section b. In this example, the area of the release liner 4 in the body section b is larger than the area of the adhesive patch. The portion 4' of the release liner 4 is not provided with the adhesive patch.
Fig. 2 is a plan view of another specific example of the skin patch sheet according to the present invention. In this example, the area of the release liner 4 in the grip section a and the body section b is larger than the area of the adhesive patch. The portion 4' of the release liner 4 is not provided with the adhesive patch.
Fig. 3 is a cross-sectional view of the skin patch sheet of Fig. 2. A carrier sheet 1, a backing 2, an adhesive layer 3 and a release liner 4 are provided in this order.
Fig. 4 is a plan view of a specific example in which a skin patch sheet is formed as plural sheets of adhesive patches having a carrier sheet 1 laminated thereon are arranged in parallel on the release liner 4. The adhesive patch is not provided at the portion 4' of the release liner 4.
Fig. 5 is a plan view of another specific example in which a skin patch sheet is formed as plural sheets of adhesive patches having a carrier sheet 1 laminated thereon are arranged in parallel on the release liner 4. The adhesive patch is not provided at the portion 4' of the release liner 4.

### Description of Embodiments

### 1. Layer configuration of skin patch sheet

The skin patch sheet of the present invention is a skin patch sheet having a carrier sheet, an adhesive patch, and a release liner laminated in this order, in which:
(a) the adhesive patch includes two layers, namely, a backing and an adhesive layer having the same shape;
(b) the carrier sheet is adjacent to the backing of the adhesive patch;
(c) the skin patch sheet includes a grip section and a body section;
(d) the area of the release liner in the body section is larger than the area of the adhesive patch;
(e) the boundary between the grip section and the body section is a slit line formed by a slit; and
(f) the slit extends from the side of the release liner, penetrating into the adhesive patch, up to a depth in the range of greater than or equal to 20% and less than 50% of the thickness on the adhesive patch side of the carrier sheet.

That is, the skin patch sheet of the present invention has a structure in which a carrier sheet, an adhesive patch, and a release liner are laminated in this order, while the adhesive patch includes two layers, namely, a backing and an adhesive layer having the same shape, and the carrier sheet is adjacent to the backing of the adhesive patch. Therefore, the skin patch sheet of the present invention has a structure in which a carrier sheet 1, a backing 2, an adhesive layer 3, and a release liner 4 are laminated in this order.

The skin patch sheet of the present invention is a skin patch sheet which is used such that while an adhesive patch including a backing and an adhesive layer is in a united state, the adhesive layer of the adhesive patch is attached to an object to be attached such as the skin surface.

The skin patch sheet of the present invention includes a grip section and a body section.

In regard to the skin patch sheet of the present invention, the "grip section" refers to a section that is used, when the adhesive patch of the skin patch sheet is attached to an object to be attached such as a human skin surface, to bring the adhesive layer of the adhesive patch of the "body section" of the skin patch sheet, which is the remainder of the skin patch sheet, into contact with the object to be attached while the grip section is gripped. The boundary between the grip section and the body section is a slit line formed by the slit that will be described in detail below. Usually, the area of the grip section is smaller as compared with the area of the body section.

### 2. Carrier sheet

In the present invention, in order to enhance operability at the time of attachment of the skin patch sheet, a carrier sheet having higher rigidity than the backing is provisionally attached in an easily detachable state, on the back surface of the backing, that is, on the surface of the backing on the reverse side of the adhesive layer.

The carrier sheet used in the present invention is provisionally attached to the back surface of the backing of the adhesive patch until the adhesive patch which includes a backing and an adhesive layer is attached to an object to be attached, and is provisionally attached in an easily detachable state so that after the adhesive patch is attached to the object to be attached such as skin, the carrier sheet can be detached from the backing.

The provisional attachment of the carrier sheet and the backing is usually carried out according to a pressurization method or a heat pressing method, both of which do not use an adhesive, but may also be carried out according to any method such as a method of interposing a weakly tacky adhesive such as polyurethane or a pressing method of using a binder such as natural rubber.

It is necessary that the peeling force between the carrier sheet and the backing be definitely smaller than the adhesive force between the adhesive layer and the skin, so that when the carrier sheet is detached from the backing, the adhesive patch attached to the object to be attached such as skin does not detach. In addition, it is preferable that the peeling force between the carrier sheet and the backing be larger than the peeling force between the adhesive layer and the release liner, so that during the operation of attachment, the process of detaching the release liner from the adhesive layer and thereby exposing the adhesive layer is not interrupted.

The material of the carrier sheet is not particularly limited as long as the material has self-supportability, and various plastic sheets and films of polyesters such as polyethylene terephthalate or polyolefins such as polyethylene and polypropylene, or kraft paper can be employed. In the case where the carrier sheet employs paper as the material, the carrier sheet may have defective appearance as a skin patch sheet by absorbing moisture and curling, or the like. Therefore, plastic laminated paper having polyethylene or the like laminated on kraft paper, high-quality paper or the like (laminate of a plastic film and paper) and the like are preferably used. The carrier sheet is more preferably a plastic sheet or film made of a polyester material having rigidity. Regarding the thickness of the carrier sheet, it is preferable to use a carrier sheet having a thickness of about 20 to 200 µm in view of handleability, and the thickness is more preferably 40 to 170 µm, even more preferably 60 to 150 µm, and particularly preferably 80 to 120 µm. The method of provisionally attaching the carrier sheet and the backing can be carried out according to a method of forming a polyurethane film that serves as the backing, on a carrier sheet that has been subjected to a peeling treatment or the like, as well as arbitrary methods such as a method of interposing a weakly tacky adhesive of polyurethane or the like, a pressurizing method, a hot pressing method, and a pressing method using a binder such as natural rubber.

In the present invention, the term "provisional attachment" means that the backing and the carrier sheet adhere with a strength weak to the extent that the carrier sheet can be separated from the backing with the force of one hand.

Since the carrier sheet is to be laminated on the backing of the adhesive patch, the carrier sheet is usually cut into the same shape and size as those of the backing, but a carrier sheet that is larger than the backing can also be used. Usually, one sheet of the carrier sheet is used in an adhesive patch. The carrier sheet can also be laminated on the backing after being partitioned; however, it is not preferable because the series of operations needed until the adhesive patch is attached to an object to be attached become complicated, and the number of waste materials generated increases.

Note that, as will be described below, when the grip section is composed only of a carrier sheet without the adhesive patch and the release liner, the shape and size of the carrier sheet will be different from those of the backing.

### 3. Backing

In the skin patch sheet of the present invention, as the backing that constitutes the adhesive patch together with the adhesive layer, a backing having a thickness of usually 20 µm or less, preferably 1 to 15 µm, more preferably 1 to 10 µm, even more preferably greater than or equal to 1 µm and less than 10 µm, particularly preferably 1 to 8 µm, and most preferably 1 to 6 µm, is employed. If the thickness of the backing is less than 1 µm, the strength required from the backing is insufficient, and the backing may be broken at the time of operation such as attaching the adhesive patch to an object to be attached, or detaching the adhesive patch from an object to be attached. If the thickness of the backing is excessively large, even if the overall thickness of the adhesive patch is made thin, it is difficult for the adhesive patch to adhere closely along the skin surface having fine surface unevenness such as sulcus cutis, and the state of attachment becomes highly noticeable, so that the sense of discomfort is also increased.

Regarding the backing, an opaque backing such as paper, a woven fabric or a nonwoven fabric can also be used. However, when it is required that after the adhesive patch is attached to the skin, the adhesive patch remains unnoticeable (cosmetic applications, transdermal absorbent applications, and the like), transparent films formed from various plastic or elastomer materials such as polyethylene, polypropylene, polyurethane, polyamide, an ethylene/vinyl acetate copolymer, a polyether, and a polyester are preferred, and particularly an elastomer film is preferred. Furthermore, it is preferable that the material of the backing do not swell in water.

Examples of the elastomer include a polyurethane elastomer, a 1,2-polybutadiene-based thermoplastic elastomer, a polystyrene-based thermoplastic elastomer, a polyolefin-based thermoplastic elastomer, and mixtures of two or more kinds thereof. Among these elastomers, from the viewpoint that the film-forming property of thin films is excellent and stretch property of the film is excellent, a polyurethane elastomer is preferred.

Examples of commercially available products of the polyurethane elastomer that is appropriate for the present invention include, but are not limited to, trade name: LUCKSKIN (registered trademark) US2268 (polyether type); LUCKSKIN U-1223, U-1285, and U-2860 (polyester type) manufactured by Seikoh Chemicals Co., Ltd. These polyurethane elastomers can be used singly, or two or more kinds thereof can be used in combination.

The glass transition temperature (Tg) of the elastomer is not particularly limited. For example, the glass transition temperature of a highly elastic polyurethane elastomer may be as high as 50°C. The glass transition temperature of the elastomer is, from the viewpoints of stretch property, flexibility, 10% tensile load, and the like of the elastomer film, preferably in the range of -70°C to 20°C, more preferably in the range of -60°C to 10°C, and particularly preferably in the range of -55°C to 0°C. Note that, the glass transition temperature of the elastomer is a value measured according to a conventional method by using a dynamic viscoelasticity measuring apparatus.

The backing in the adhesive patch of the skin patch sheet of the present invention is preferably an elastomer film having a thickness of greater than or equal to 1 µm and less than 10 µm. The elastomer film preferably has a smaller difference in terms of the direction of properties (anisotropy), from the viewpoint of reducing the sense of discomfort during attachment (meaning the sensation of resistance of the adhesive patch perceived when the skin expands or contracts). The method for forming the elastomer film is preferably a solution casting method, since a thin film having substantially the same properties such as 10% tensile load in the longitudinal direction and the transverse direction can be obtained. However, there is little chance that the 10% tensile load value of the elastomer film varies to a large extent in the longitudinal direction and the transverse direction, even if other film-forming methods are used.

The thickness of the elastomer film that constitutes the backing is even more preferably in the range of 1 to 9 µm, and more preferably in the range of 1 to 8 µm. From the viewpoints of making the adhesive patch hardly noticeable or mitigating the sense of discomfort during attachment, the thickness of the elastomer film can be reduced to the range of 1 to 6 µm. If the thickness of the elastomer film is less than 1 µm, film forming may become difficult.

In order to control the 10% tensile load in the longitudinal direction and the transverse direction of the adhesive patch respectively to the range of 0.01 to 1.2 N/10 mm, it is preferable to use, as the backing, an elastomer film having 10% tensile loads in the longitudinal direction and the transverse direction as measured according to the Japanese Industrial Standards JIS Z 0237, respectively in the range of 0.01 to 1.2 N/10 mm. The 10% tensile loads in the longitudinal direction and the transverse direction of the elastomer film are preferably respectively in the range of 0.03 to 1.1 N/10 mm, more preferably respectively in the range of 0.05 to 1.0 N/10 mm, and particularly preferably respectively in the range of 0.06 to 0.95 N/10 mm.

Since the thickness of an elastomer film having an excessively small 10% tensile load generally becomes too small, the film-forming property or handleability deteriorates. If the 10% tensile load of the elastomer film is excessively large, rigidity increases, and elasticity or flexibility becomes insufficient. Therefore, it is difficult for an adhesive patch employing such elastomer film as the backing, to adhere along the skin surface having fine surface unevenness such as sulcus cutis, and the state of attachment becomes highly noticeable. Furthermore, an adhesive patch employing an elastomer film having an excessively large 10% tensile load as the backing, gives a strong sense of discomfort at the time of attachment to the skin surface.

In this regard, a polyethylene terephthalate (PET) film is such that the 10% tensile loads in the longitudinal direction and the transverse direction respectively have a magnitude of greater than 3.0 N/10 mm even when the thickness of the film is as thin as 1.5 µm. Therefore, a polyethylene terephthalate film is not preferable as a backing that is used in the case where it is particularly necessary that the backing be unnoticeable in cosmetic applications according to the present invention. That is, since it is difficult for an adhesive patch employing a PET film as the backing, to closely adhere along the skin surface having fine surface unevenness such as sulcus cutis, the state of attachment is highly noticeable, and the sense of discomfort at the time of attachment to the skin surface is strong.

### 4. Adhesive layer

In regard to the skin patch sheet of the present invention, in the adhesive layer that constitutes the adhesive patch together with the backing, any known adhesive such as a rubber-based adhesive, an acrylic adhesive, a urethane-based adhesive, a silicone-based adhesive or a vinyl ether-based adhesive can be used. Regarding the adhesive layer, when the object to be attached is the skin surface, an adhesive having low skin irritability is employed, and an acrylic adhesive or a silicone-based adhesive is preferred. Furthermore, when a retention power is required such as in the case where the adhesive patch is securely attached to the skin to restrain the skin, an acrylic adhesive is preferred, and when it is necessary to suppress the amount of keratin detachment to a low level, a urethane-based adhesive or an addition reaction type silicone adhesive is particularly preferred.

The thickness of the adhesive layer is usually in the range of 1 to 25 µm, preferably 2 to 22 µm, more preferably 3 to 20 µm, even more preferably 3 to 15 µm, and particularly preferably 4 to 12 µm.

### [Pharmacologically active substance]

The adhesive layer can have a pharmacologically active substance incorporated therein. Therefore, in the present invention, the adhesive patch may include either an adhesive layer containing a pharmacologically active substance, or an adhesive layer which does not contain a pharmacologically active substance.

There are no particular limitations on the pharmacologically active substance, but ceramides; vitamins that are effective in moisturization of skin, prevention of the occurrence of wrinkles or freckles, and the like; glycyrrhetinic acid, glycyrrhizinic acid salts and menthol, which suppress inflammation of skin; crotamiton that suppresses itchiness of skin; lidocaine as an analgesic; and the like can be used.

Regarding the vitamins, at least one selected from the group consisting of vitamin E or esters thereof, which are fat-soluble vitamins; vitamin B6, vitamin C and esters thereof, which are water-soluble vitamins; and derivatives thereof is effective. Particularly preferred examples of the pharmacologically active substance include ascorbyl tetrahexyldecanoate [trade name: NIKKOL (registered trademark) VC-IP, Nikko Chemicals Co., Ltd.], which is a vitamin C derivative that has high penetrability to the skin, alleviates oxidation stress, and suppresses pigment deposition, and pyridoxine tri-2-hexyldecanoate (trade name: NIKKOL VB6-IP, Nikko Chemicals Co., Ltd.), which is a vitamin B6 derivative having a high moisture-retaining effect.

Regarding the pharmacologically active substance, addition of ceramide 1 that is considered to undergo significant amount reduction at the affected sites of atopic dermatitis; phytosphingosine that accelerates the synthesis of ceramides and has a bacteriostatic action; and the like is also useful. Furthermore, moisturizers such as glycerin, propylene glycol, sorbitol, 1,3-butylene glycol, polyethylene glycol, urea, sodium lactate, peptides, heparin, and hyaluronic acid for appropriately maintaining the moisture retaining property, can also be added. In addition, components that are expected to be effective for various skin troubles such as dark spots are also useful as a pharmacologically active substance to be added. Furthermore, it is preferable to incorporate antibacterial agents and the like as necessary, since proliferation of contaminating bacteria at the site of the object to which the adhesive patch has been attached can be prevented.

### 5. Adhesive patch

The adhesive patch in the skin patch sheet of the present invention is a patch formed by two layers, namely, a backing and an adhesive layer having the same shape laminated together.

The total thickness of the backing and the adhesive layer (may be simply referred to as the "thickness of the adhesive patch") is usually in the range of 2 to 25 µm, preferably 3 to 20 µm, and more preferably 4 to 18 µm. In many cases, satisfactory results can be brought about with a thickness of the adhesive patch in the range of as small as 4 to 14 µm.

If the thickness of the adhesive patch is too small, the adhesive power may deteriorate, or production may become difficult. If the thickness of the adhesive patch is too large, the adhesive patch after being attached becomes highly noticeable, and the sense of discomfort at the time of attachment may increase.

The size and shape of the adhesive patch may be any size and any shape that are usually employed for adhesive patches, and there are no particular limitations; however, an adhesive patch having an approximately rectangular shape having short edges and long edges is easy to handle. Furthermore, a so-called capsule-like shape in which the vicinities of the four vertexes of the approximately rectangular shape are cut out to an approximately arc shape and rounded (having rounded corners), is preferred. Particularly, in the case of attaching the adhesive patch to the face, an approximately rectangular shape in which, when a longer edge is designated as the longitudinal direction and a shorter edge is designated as the transverse direction, the length in the longitudinal direction is in the range of 2.0 to 8.0 cm, preferably 3.0 to 7.0 cm, and more preferably 3.5 to 6.0 cm, and the length in the transverse direction is in the range of 0.5 to 3.5 cm, preferably 0.8 to 3.0 cm, and more preferably 1.0 to 2.5 cm, and a capsule-like shape having rounded corners in the vicinity of the vertexes, are preferred. The ratio of the lengths of a shorter edge and a longer edge of the adhesive patch is preferably in the range of 1:2 to 1:9, and more preferably in the range of 1:3 to 1:6.

Examples of the shape of the adhesive patch that can be used include, in addition to the capsule-like shape, a teardrop shape (droplet shape), a half-moon shape, a crescent shape, a gourd shape, a circular shape, and a boomerang shape.

### 6. Release liner

The release liner in the skin patch sheet of the present invention is to coat and protect the adhesive layer in order to prevent the surface of the adhesive layer of the adhesive patch from being contaminated before attachment, and the release liner is provisionally attached to the adhesive layer in an easily detachable state. In regard to the extent of the easily detachable state, the peeling force between the release liner and the adhesive layer is preferably smaller than the peeling force between the carrier sheet and the backing, so that the process of detaching the release liner from the adhesive layer and thereby exposing the adhesive layer is not interrupted.

Regarding the release liner, usually, use can be made of those release liners that are generally used in the art of adhesive patches, such as release liners produced by subjecting a surface of a plastic sheet or film of a polyolefin such as polyethylene or polypropylene, or a polyester represented by polyethylene terephthalate, a laminate of a plastic film and paper (plastic laminated paper), and the like, to a silicone release treatment. Furthermore, since there is a risk that the pharmacologically active substance contained in the adhesive layer may migrate to the release liner, similarly to the carrier sheet, it is preferable to process a non-drug adsorptive film such as a polyester film by a surface treatment. The thickness of the release liner can be appropriately set, but from the viewpoint of handleability, the thickness is usually in the range of 10 to 500 µm, preferably 20 to 300 µm, and more preferably 25 to 200 µm.

### [Area of release liner]

The skin patch sheet of the present invention is such that at least in the body section, the area of the release liner is larger than the area of the adhesive patch, that is, the area of the backing and the adhesive layer.

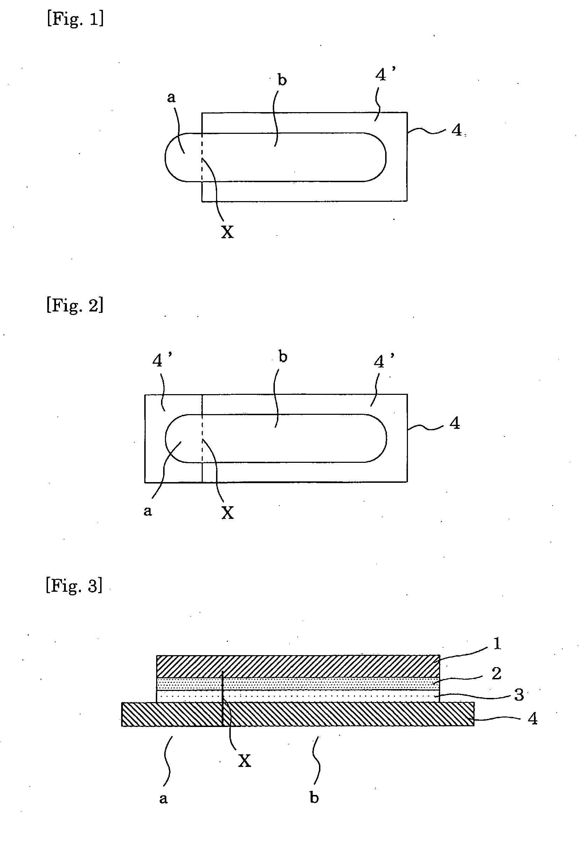

In the body section, since the release liner is larger than the adhesive patch, when the grip section of the skin patch sheet is held by using the fingers of one hand, the release liner of the portion where the adhesive patch of the skin patch sheet is not provided (portion 4' in Fig. 1 and Fig. 2) is pressed with the same hand or the other hand, and the grip section is detached to the carrier sheet side, the adhesive patch can be easily detached from the release liner.

On the other hand, in the body section, if the area of the release liner is equal to or less than the area of the adhesive patch, when the release liner is detached from the adhesive patch, there is a need to provide a clue to detach the release liner from the partition line by bending the entire skin patch sheet to a certain degree. However, for example, in the case of a very thin backing having a thickness of greater than or equal to 1 µm and less than 10 µm, the carrier sheet does not have high rigidity, and it is difficult to take a chance to detach the backing. Particularly, when a material having low rigidity, such as paper, is used in the carrier sheet, even if the skin patch sheet is bent, the release liner may not be securely detached from the partition line. Furthermore, when the skin patch sheet is bent several times in order to detach the release liner, unnecessary force is exerted to the grip section, and there is an inconvenience that the grip section may be torn off before the adhesive patch is detached from the release liner.

The skin patch sheet of the present invention is such that in regard to the grip section, the area of the release liner may not be larger than the area of the adhesive patch. For example, as illustrated in Fig. 1, in the grip section, the release liner and the adhesive patch may have the same shape, and in this case, the outer periphery (outline) of the grip section of the skin patch sheet is formed by a slit and is formed by being connected to a slit line that serves as a boundary between the grip section and the body section. The grip section in which the release liner and the adhesive patch have the same shape, can be easily formed by a punching tool having a shape matching the shape of the grip section, while having a carrier sheet, an adhesive patch and a release liner laminated together.

Furthermore, as illustrated in Fig. 2, the skin patch sheet of the present invention is such that in addition to the body section, even in the grip section, the area of the release liner may be larger than the area of the adhesive patch, that is, the area of the backing and the adhesive layer. In this case, the adhesive patch and the carrier sheet provided in the grip section are held, the adhesive patch and the carrier sheet provided in the grip section and the body section are separated from the release liner and then attached to an object to be attached such as skin.

Note that, in the present invention, when it is said that the area of the release liner is larger than the area of the adhesive patch, it is implied that the area of the release liner is larger than the area of the adhesive patch, usually by 15% or more, preferably 20% or more, and more preferably 25% or more. If the difference of the area is less than 15%, the effects of the present invention may not be sufficiently exhibited. Furthermore, particularly, in the case of a skin patch sheet in which plural sheets of adhesive patches that will be described below are arranged in parallel on the release liner, there may be an inconvenience that the adhesive patches should be arranged without gaps.

### 7. Grip section

The shape of the grip section of the skin patch sheet of the present invention is not particularly limited, and a circular shape, a rectangular shape, an elliptical shape, a chevron shape, and the like can be used. However, it is convenient in view of production process, to adopt a shape corresponding to the shape of the adhesive patch described above, such as an approximately rectangular shape or a capsule shape, as the shape of the grip section.

Furthermore, the grip section usually includes a carrier sheet, an adhesive patch and a release liner, but the carrier sheet is the only essential component. For example, the grip section may also be composed only of a carrier sheet, excluding the adhesive patch (including a backing and an adhesive layer) and the release liner.

Furthermore, as a device to facilitate gripping of the grip section, reinforcement for the grip section may be further added to the surface where the adhesive patch of the grip section is disposed, or to the surface on the reverse side. For example, an adhesive tape having an area slightly larger than that of the grip section can be laminated so as to increase rigidity of the grip section.

### 8. Slit

The skin patch sheet of the present invention is such that the boundary between the grip section and the body section is a slit line formed by a characteristic slit. This slit extends from the release liner side, penetrating into the release liner and the adhesive patch, up to a depth in the range of greater than or equal to 20% and less than 50% of the thickness on the adhesive patch side of the carrier sheet.

That is, the slit that forms the slit line partitions the release liner, the adhesive layer and the backing by penetrating into these, and also, the carrier sheet is cut such that at least a half or more of the thickness of the carrier sheet is left. When the carrier sheet is cut to a depth in the range of 50% or more of the thickness on the adhesive patch side of the carrier sheet, the strength of the area of the carrier sheet where the slit is present is reduced. Therefore, there is a risk that there may be an inconvenience that the grip section is torn off when the adhesive patch is attached to an object to be attached, or before the adhesive patch is detached from the release liner. The depth of the slit on the adhesive patch side of the carrier sheet is preferably in the range of 25 to 45%, and more preferably 30 to 40%, of the thickness of the carrier sheet.

In the skin patch sheet of the present invention, when this slit is provided, the carrier sheet in the grip section (including the case where an adhesive patch and a carrier sheet are laminated) may form, under no load, an angle of 1 to 90°, preferably an angle of 3 to 80°, and more preferably 5 to 70°, with respect to the extended direction of the surface of the body section, with the tip of the slit serving as the fulcrum. In this case, since the carrier sheet in the grip section is lifted up at the tear toward the side where the adhesive patch is detached from the release liner, the adhesive patch can be more easily detached, and visibility is also satisfactory, so it is preferable. As a result, when the adhesive patch is detached from the release liner in the body section of the skin patch sheet, there is no risk that the surface of the adhesive may be contaminated, and the adhesive patch can be neatly attached to an object to be attached such as skin. Thus, the operation of attachment of the adhesive patch to an object to be attached is made easier. The angle of the carrier sheet in the grip section with respect to the extended direction of the body section can be adjusted by varying the position or length of the slit, the thickness of the carrier sheet, and the like.

The slit line is usually formed by a slit that is cut at a position separated apart from the end of the longer edge direction of the adhesive patch on the grip section by a distance in the range of 1/10 to 2/5, and preferably 1/8 to 1/3, of the length of the longer edge, straight in the shorter edge direction of the adhesive patch (perpendicular direction). If the slit line is provided at a position too close to the end of the longer edge direction of the adhesive patch on the grip section, the strength of the grip section may be insufficient, and the adhesive may be easily touched by the hand. If the slit line is provided at a position too distant from the end of the longer edge direction of the adhesive patch on the grip section, the amount of waste that is finally disposed of from the skin patch sheet increases.

In addition to the straight line, the slit line may be a curve or may be a zigzag line. Specifically, a shape such as an arc shape, a wave shape or a saw-toothed shape can also be used. At the tip of the slit, a V-shaped or U-shaped notch or depression may also be provided in the shorter edge direction of the adhesive patch. The length of the notch or depression is usually preferably set to one-half or less of the length of the shorter edge direction.

### 9. Arrangement of plural adhesive patches

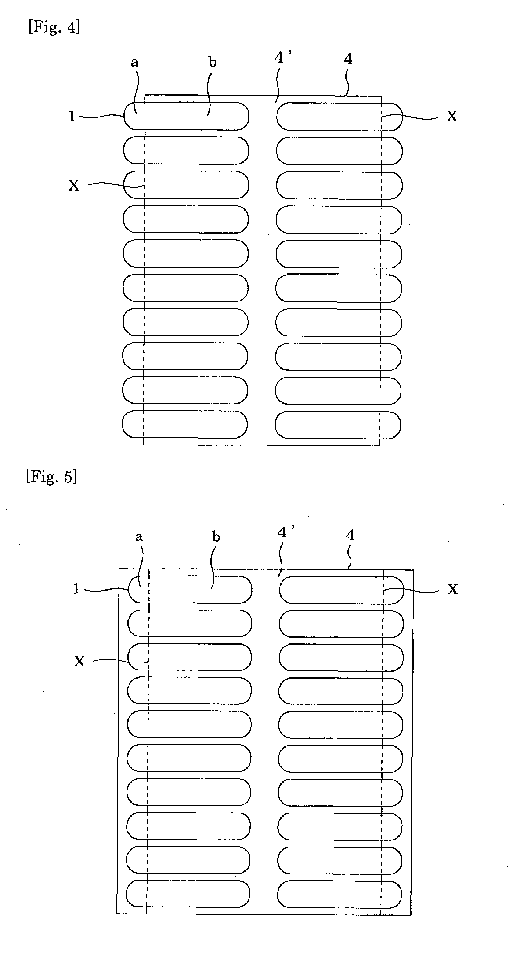

The skin patch sheet of the present invention can be used as a simple form in which one each of a carrier sheet, an adhesive patch and a release liner are provided; however, as illustrated in Figs. 4 and 5, a skin patch sheet in which plural sheets of adhesive patches laminated with a carrier sheet are formed in parallel on one release liner, may also be used. In this case, the release liner is shared by the plural sheets of adhesive patches on which the carrier sheet is laminated. When a skin patch sheet is formed such that plural sheets of the adhesive patches each having a carrier sheet laminated thereon are arranged in multiple lines on the release liner, the operation of detaching the adhesive patch from the release liner and attaching the adhesive patch to an object to be attached such as skin, can be carried out sequentially with one hand, and therefore, operability is enhanced.

When a skin patch sheet is formed such that plural sheets of adhesive patches each having a carrier sheet laminated thereon are arranged in parallel on a release liner, the adhesive patches may be arrayed without any gaps on the release liner, or may be arrayed apart from one another at a certain distance. However, if the adhesive patches are arrayed without gaps, depending on the method of detaching adjoining adhesive patches, there may be an inconvenience that air may be incorporated into neighboring adhesive patches on the release liner, or creases may be generated.

In the case where plural sheets of adhesive patches each having a carrier sheet laminated thereon are arrayed without any gaps on the release liner, it is often needed to contrive a device such as further providing a slit that completely divides the carrier sheet, between two adjoining adhesive sheets from the grip section side. Therefore, it is preferable that plural sheets of adhesive patches on which the carrier sheet is laminated be arrayed apart from one another at a certain interval on the release liner, and the adhesive patches are arranged at an interval of usually about 1 to 10 mm, more preferably about 1.5 to 8 mm, and even more preferably about 2 to 7 mm.

### 10. Method for producing skin patch sheet

The method for producing the skin patch sheet of the present invention is not particularly limited, if a skin patch sheet having a carrier sheet 1, an adhesive patch (including two layers, namely, a backing 2 and an adhesive layer 3 having the same shape) and a release liner 4 laminated in this order can be produced, and a slit line which is a boundary between a grip section a and a body section b, can be formed by a slit X that extends from the release liner side, penetrating into the adhesive patch, up to a depth in the range of greater than or equal to 20% and less than 50% of the thickness in the adhesive patch side of the carrier sheet 1. Hereinafter, an example of the method for producing a skin patch sheet by arranging plural sheets of adhesive patches each having a carrier sheet laminated thereon, in parallel on a release liner, will be described.

An organic solvent solution of an adhesive such as an acrylic adhesive or a silicone-based adhesive is applied on one surface of a release liner 4 to a predetermined thickness by a bar coating method, and then the solution is dried to form an adhesive layer 3.

On the other hand, a polyester film, a polyolefin film, or a plastic laminated paper obtained by laminating polyethylene or the like on high-quality paper or the like is used as a carrier sheet 1. As for the case of a plastic laminated paper, a polyether type or polyester type polyurethane elastomer solution or the like is applied on the surface of the polyethylene side or the like to a predetermined thickness by a bar coating method, and the solution is dried. Thus, a backing 2 is produced. As the polyether type polyurethane elastomer solution, trade name "LUCKSKIN US2268" (Tg = -23.1°C) manufactured by Seikoh Chemicals Co., Ltd. or the like can be used.

The backing 2 and the adhesive layer 3 obtained by the method described above are superimposed, and thus a skin patch sheet having a laminate configuration of carrier sheet 1/backing 2/adhesive layer 3/release liner 4 is produced. Herein, a method for producing a skin patch sheet by a transfer coating method has been described, but the present invention is not intended to be limited thereto, and the skin patch sheet may also be produced by various coating methods such as comma coating, knife coating, and reverse coating. Furthermore, regarding the coating method, a direct coating method or the like can also be used.

Thereafter, at a position distant by, for example, about 1 cm from the end of the longer edge direction of the adhesive patch having an approximately rectangular shape, a slit X is inserted which extends from the release liner 4 side, penetrating into the adhesive layer 3 and the backing 2, up to a depth in the range of greater than or equal to 20% and less than 50% of the thickness on the adhesive patch side of the carrier sheet 1.

Subsequently, cutting (for example, reciprocating type or rotary type) of the carrier sheet 1 and the adhesive patch is carried out from the side of the carrier sheet 1 toward the side of the release liner 4, such that twenty adhesive patches are arranged in parallel on the release liner 4 having a size of 12 cm in width and 10 cm in length, while the adhesive patch is cut to a rectangular shape having a size of, for example, 1 cm in width and 5 cm in length, and the vicinities of the vertexes are rounded (having rounded corners). At this time, in the area that forms a grip section a, a cut which penetrates from the release liner 4 to the carrier sheet 1 is introduced, and a skin patch sheet is produced by remaining peripheral corners of the grip section a. Thereafter, the skin patch sheet may be put to use by taking off the marginal areas (carrier sheet 1, backing 2 and adhesive layer 3) other than the adhesive patch and the corner areas (carrier sheet 1, backing 2, adhesive layer 3 and release liner 4).

Furthermore, as another aspect and production method for the skin patch sheet, although the number of processes increases, a skin patch sheet may also be obtained by producing an original roll of a roll-shaped adhesive tape having a laminate configuration of carrier sheet/backing/adhesive layer/release liner; forming one or plural slits which each extend from the release liner side, penetrating into the adhesive patch (backing and adhesive layer), up to a depth in the range of greater than or equal to 20% and less than 50% of the thickness on the adhesive patch side of the carrier sheet; completely detaching the release liner that is divided into two or more parts at the slit; laminating on the adhesive layer a release liner having a size sufficiently large to cover the area of slit of the adhesive surface thus exposed; subsequently cutting the skin patch sheet into a desired shape from the carrier sheet side; excluding the areas of carrier sheet/backing/adhesive layer which are unnecessary constituents other than the skin patch sheet; and then cutting the release liner such that one sheet or plural sheets of adhesive patches each having a carrier sheet laminated on the release liner are mounted.

The skin patch sheet obtainable in this case is a skin patch sheet in which one sheet or plural sheets of adhesive patches having a carrier sheet laminated thereon are formed on one release liner. Therefore, in the skin patch sheet of the present invention, the phrase "(f) the slit extends from the release liner side, penetrating into the adhesive patch, up to a depth in the range of greater than or equal to 20% and less than 50% of the thickness on the adhesive patch side of the carrier sheet" implies that the slit does not penetrate into the release liner. Also in this aspect, the grip section may be composed only of a carrier sheet without the adhesive patch (including a backing and an adhesive layer) and a release liner.

Furthermore, as another aspect of the skin patch sheet, a two-sided tape for fixing the skin patch sheet to a table or the like can be disposed over the entire surface or a portion of the surface of the release liner opposite to the adhesive patch.

The skin patch sheet is supplied in a state of being placed in a package that is usually used by those skilled in the art.

### 11. Procedure of attachment of adhesive patch to object to be attached

The operation procedure of attaching the adhesive patch to an object to be attached such as skin will be explained by using the skin patch sheet of the present invention.

First, the grip section a of the skin patch sheet is held to make the carrier sheet 1 to face upward, the adhesive patch is detached from the release liner 4, and the adhesive at the surface of the adhesive layer 3 is exposed. The grip section a of the skin patch sheet is such that since there is a slit X which extends to a depth in the range of greater than or equal to 20% and less than 50% of the thickness on the adhesive patch side in the carrier sheet 1, the grip section a may be in a state of being bent by 1 to 90° with respect to the extended direction of the surface of the body section b on the carrier sheet side. In this case, visibility of the grip section a is good, and the adhesive patch can be easily detached from the release liner 4 even with one hand.

Next, the adhesive patch is attached such that the surface of the adhesive layer 3 faces an object to be attached such as skin. At the time of attaching the adhesive patch, since the carrier sheet 1 is provisionally attached on the backing 2, even if a backing 2 having a small thickness is used, the adhesive patch has self-supportability. Therefore, the adhesive patch can be attached to the skin without causing creases in the backing 2, or having the adhesive sticking to the finger.

After the attachment of the adhesive patch, since the adhesive layer 3 and the backing 2 are divided by the slit X into the grip section a and the body section b, when the grip section a is detached, only the adhesive patch of the body section b that includes the backing 2 and the adhesive layer 3 is attached to the skin, and the operation of attachment is finished.

Through such a procedure, a series of operations of detachment of the adhesive patch from the release liner, attachment of the adhesive patch to the skin, and completing attachment by detaching the carrier sheet including the grip section, can be carried out even with one hand. Furthermore, there are advantages that waste materials can be disposed of at one time, and substantially only one waste material is generated in one operation of attachment.

### 12. Applications of skin patch sheet, etc.

The skin patch sheet of the present invention can be utilized as various adhesive patches for medical applications, cosmetic applications, industrial applications, stationery applications, domestic applications, and the like.

For example, an adhesive patch having a capsule-like shape in which a thin polyurethane elastomer film having a thickness of 1 to 10 µm is used as a backing, and a relatively thin adhesive layer containing an acrylic or silicone-based adhesive and having a thickness of 3 to 8 µm is laminated thereon, and which has an approximately rectangular shape having a size of 1 to 3 cm in width × 3 to 5 cm in length, with the vicinities of vertexes being rounded, is suitable to be used for cosmetic applications, particularly for a so-called lift-up application by which wrinkles or sagging is lifted up and extended, by attaching the adhesive patch around the eyes, behind the ears or on the cheeks in the face. As for the skin patch sheet of the present invention, since the adhesive patch can be attached by using one hand, in the lift-up application, the operation of attaching the adhesive patch may be carried out with one hand while suppressing the skin of the face with the other hand. Therefore, it is easy for a user to perform the operation of implementing lift-up by herself/himself, and the skin patch sheet has enhanced handleability as compared with conventional adhesive patches.

Furthermore, the skin patch sheet of the present invention can avoid aggravation of an affected site caused by scratching and can accelerate natural recovery at an affected site of dermatitis such as atopic dermatitis, by covering the affected site of the skin. That is, the skin patch sheet of the present invention is such that since a polyurethane elastomer film having a small thickness or the like can be used as the backing, regardless of whether the adhesive layer contains a pharmacologically active substance or not, the skin patch sheet can protect the skin surface with appropriate moisture permeability. As a result, the skin patch sheet of the present invention can prevent the penetration of foreign materials, and can prevent a person having the symptoms of dermatitis from unconsciously scratching the skin. Furthermore, the skin patch sheet of the present invention is such that even in the case where a person having the symptoms of atopic dermatitis takes an action of scratching the skin, since the adhesive patch on the skin functions as a barrier that prevents direct scratching of the skin, the skin patch sheet is also useful as an adhesive patch not only for medical applications but also for domestic or cosmetic applications.

### EXAMPLES

Hereinafter, the present invention will be more specifically described by way of Examples and Comparative Examples, but the present invention is not intended to be limited to these Examples. The shape of the skin patch sheet and the methods for measuring the attachment characteristics in Examples and Comparative Examples are as follows.

### (1) Thickness of layers

The thicknesses of the carrier sheet, backing, adhesive layer and release liner were measured with a dial thickness gauge.

### (2) Depth of slit

The depth of the slit was measured by using a laser microscope.

### (3) Evaluation of attachment characteristics

In regard to the evaluation of the attachment characteristics of the skin patch sheet of the following terms 1) to 3), a sensory evaluation was carried out by using the skin patch sheets produced in Examples and Comparative Examples, on the usability of the skin patch sheets against human skin until the attachment of the adhesive patch was exhausted. Specifically, five male and female adults in their 20's and 30's were selected as testees, and each testee attached 5 sheets of the adhesive patches having a size of 1 cm in width and 4.5 cm in length, which were detached from the release liner, on the inner part of the forearm. At the time of attaching, the adhesive of the adhesive patches was attached to the skin and then lightly pressed with the finger.

### 1) Evaluation upon detachment from release liner

Five sheets of the aforementioned adhesive patches were detached from the release liner, and an evaluation was conducted on the detachment of the adhesive patches from the release liner. The evaluation criteria are as follows.
AA: Four to five people out of the 5 testees answered that there were no weight or strength required to detach, creases of the adhesive patches, and tape residue on the release liner.
A: Three people out of the 5 testees answered that there were no weight or strength required to detach, creases of the adhesive patches, and tape residue on the release liner.
B: Two people out of the 5 testees answered that there were no weight or strength required to detach, creases of the adhesive patches, and tape residue on the release liner.
C: One or zero people out of the 5 testees answered that there were no weight or strength required to detach, creases of the adhesive patches, and tape residue on the release liner.

### 2) Evaluation upon attachment (contact to adhesive)

An evaluation was conducted on the presence or absence of contact to the adhesive when five sheets of the aforementioned adhesive patches that had been detached from the release liner, were attached on the inner part of the forearm. When the finger touched the adhesive even in one sheet out of the five sheets, it was considered that "the finger touched the adhesive". The evaluation criteria are as follows.
AA: Five people out of the 5 testees could attach the adhesive patches without the finger touching the adhesive until the time point of attaching the adhesive patches to the skin.
A: Three or four people out of the 5 testees could attach the adhesive patches without the finger touching the adhesive until the time point of attaching the adhesive patches to the skin.
B: One or two people out of the 5 testees could attach the adhesive patches without the finger touching the adhesive until the time point of attaching the adhesive patches to the skin.
C: Five people out of the 5 testees experienced the finger touching the adhesive until the time point of attaching the adhesive patches to the skin.

### 3) Evaluation of handleability

An evaluation was conducted on a series of operations of detaching the adhesive patch from the release liner, attaching the adhesive patch to the skin, and completing the attachment by detaching the carrier sheet including the grip section. The evaluation criteria are as follows.
AA: Four or five people out of the 5 testees answered that handleability was satisfactory.
A: Three people out of the 5 testees answered that handleability was satisfactory.
B: Two people out of the 5 testees answered that handleability was satisfactory.
C: One or zero people out of the 5 testees answered that handleability was satisfactory.

### [Example 1]

A high-quality paper (thickness: 100 µm) laminated with polyethylene was used as a carrier sheet 1, and a polyether type moisture-permeable polyurethane elastomer solution was applied by a bar coating method on the surface of the polyethylene side such that the thickness after being dried would be 5 µm, and the solution was dried. Thus, a backing 2 was formed. The polyether type polyurethane elastomer solution was trade name "LUCKSKIN US2268" (Tg = -23.1°C) manufactured by Seikoh Chemicals Co., Ltd.

An organic solvent solution of an acrylic adhesive (a copolymer of 2-ethylhexyl acrylate/vinyl acetate/acrylic acid = 85/11/4 weight ratio) was applied by a bar coating method on one surface of the release liner 4 (a silicone-treated polyethylene laminated paper, length: 10 cm, width: 12 cm, thickness: 120 µm) such that the thickness after being dried would be 5 µm. Thereafter, the solution was dried, and an adhesive layer 3 was formed.

The adhesive layer 3 was superimposed on the backing 2 side of the laminate including the carrier sheet 1 and the backing 2 obtained as described above, and thus a skin patch sheet having a laminate configuration of carrier sheet 1/backing 2/adhesive layer 3/release liner 4 was produced.

A straight line-shaped slit X which extended from the release liner 4 side, penetrating into the adhesive patch (including the adhesive layer 3 and the backing 2), up to a depth of 35 µm on the adhesive patch side of the carrier sheet 1, was inserted at a position 1 cm away from the edge in the longitudinal direction of the skin patch sheet.

Subsequently, the carrier sheet 1 and the adhesive patch were cut from the side of the carrier sheet 1 toward the side of the release liner 4, such that the adhesive patch acquired a capsule-like shape with an approximately rectangular shape having a size of 1 cm in width and 4.5 cm in length, with the vicinities of vertexes being rounded (having rounded corners), and twenty adhesive patches (10 patches x 2 rows) were arranged in parallel on the release liner 4. The interval between adjoining adhesive patches was about 2 mm.

At this time, a cut that penetrated into the carrier sheet 1 was inserted from the release liner 4 side in the portion of the release liner 4 where the adhesive patches that would serve as the grip section a were not provided, and the cut was made along a line constituting a line that is 1 cm away from the edge in the longitudinal direction of the adhesive patches and the outline of the adhesive patches. Thus, a skin patch sheet in which plural sheets of the adhesive patches were arranged in parallel on the release liner 4 was produced. Thereafter, the marginal areas (carrier sheet 1, backing 2, and adhesive layer 3) other than the adhesive patches of the body section b, and the corner areas (carrier sheet 1, backing 2, adhesive layer 3, and release liner 4) of the grip section a were peeled off, and thus a skin patch sheet illustrated in Fig. 4 was produced. The results for an evaluation of the attachment characteristics of the skin patch sheet are presented in Table 1.

### [Example 2]

A skin patch sheet was produced by the same procedure as that used in Example 1, except that the grip section a was produced to be composed only of a carrier sheet 1 (the backing 2, adhesive layer 3 and release liner 4 were removed). The results for an evaluation of the attachment characteristics of the skin patch sheet are presented in Table 1.

### [Comparative Example 1]

A skin patch sheet in which all of the various layers of carrier sheet 1/backing 2/adhesive layer 3/release liner 4 had the same size, was produced in a number as many as required for the evaluation, by the same procedure as that used in Example 1, except that a slit X on the side of the release liner 4 was not inserted, and accordingly, a slit line that would serve as the boundary between the grip section and the body section was not formed, and that the release liner was produced to have the same shape and size as those of the skin patch sheet having a capsule-like shape which was an approximately rectangular shape having a size of 1 cm in width x 4.5 cm in length, with the vicinities of vertexes being rounded (having rounded corners). The results for an evaluation of the attachment characteristics of the skin patch sheet are presented in Table 1.

### [Comparative Example 2]

A skin patch sheet was produced by the same procedure as that used in Example 1, except that a slit line was formed by inserting the slit X deeply to reach a depth of 60 µm of the carrier sheet 1. The results for an evaluation of the attachment characteristics of the skin patch sheet are presented in Table 1.

**[Table 1]**

| | Example 1 | Example 2 | Comp. Ex. 1 | Comp. Ex. 2 |
|---|---|---|---|---|
| Thickness of carrier sheet (µm) | 100 | 100 | 100 | 100 |
| Depth of slit (µm) | 35 | 35 | None | 60 |
| Evaluation upon detachment from release liner | AA | AA | B | B |
| Evaluation upon attachment | AA | AA | C | AA |
| Evaluation of handleability | AA | AA | C | B |

The skin patch sheets of Examples 1 and 2, in which a slit line that was a boundary between the grip section and the body section was formed by a slit X that extended from the release liner side, penetrating into the adhesive patch, up to a depth in the range of greater than or equal to 20% and less than 50% of the thickness of the carrier sheet, were excellent in all of the evaluation upon detachment of the adhesive patches from the release liner, the evaluation upon attachment, and the evaluation of handleability. On the contrary, the skin patch sheet of Comparative Example 1, in which a slit that extended from the release liner side, penetrating into the adhesive patch, up to the carrier sheet was not formed, was satisfactory (B) or poor (C) in the evaluation upon detachment from the release liner, the evaluation upon attachment, and the evaluation of handleability. Furthermore, the skin patch sheet of Comparative Example 2, in which a slit that extended from the release liner side, penetrating into the adhesive patch, up to the carrier sheet was formed to a depth in the range of 50% or more of the thickness of the carrier sheet, did not bring about touch to the adhesive, but the results of the evaluation upon detachment from the release liner and the evaluation of handleability were merely satisfactory (B).

### INDUSTRIAL APPLICABILITY

According to the present invention, since a particular slit line which extends from the release liner to the backing is provided to an adhesive patch to which a carrier sheet having self-supportability is provisionally attached in a easily detachable state on the rear surface of the backing, the operability of attachment to an object to be attached is excellent, and the adhesive patch can be neatly attached without generating creases. Furthermore, the surface of the adhesive layer is not subject to contamination with the finger at the time of attachment, and detachment of the carrier sheet can also be carried out conveniently as compared with conventional adhesive patches.

### REFERENCE SIGNS LIST

- 1: CARRIER SHEET
- 2: BACKING
- 3: ADHESIVE LAYER
- 4: RELEASE LINER
- 4': RELEASE LINER (WITHOUT ADHESIVE PATCH)
- a: GRIP SECTION
- b: BODY SECTION
- X: SLIT

## Claims

1. A skin patch sheet comprising a carrier sheet (1), an adhesive patch and a release liner (4) laminated in this order, wherein
(a) the adhesive patch comprises two layers of a backing (2) and an adhesive layer (3) having the same shape,
(b) the carrier sheet is adjacent to the backing of the adhesive patch, **characterized in that**,
(c) the skin patch sheet comprises a grip section (a) and a body section (b),
(d) in the body section, the area of the release liner is larger than the area of the adhesive patch,
(e) the boundary between the grip section and the body section is a slit line formed by a slit (x), and
(f) the slit cuts into the skin patch sheet from the release liner side, penetrating into the adhesive patch, to a position at which at least half or more of the thickness of the carrier sheet on the adhesive patch is left uncut.

2. The skin patch sheet according to claim 1, wherein the slit extends from the release liner side, penetrating into the adhesive patch, up to a depth in the range of greater than or equal to 20% and less than 50% of the thickness of the carrier sheet on the adhesive patch side.

3. The skin patch sheet according to claim 1 or claim 2, wherein plural sheets of the adhesive patches, each having a carrier sheet laminated thereon, are arranged in parallel on the release liner; and preferably wherein the outer periphery of the grip section is formed by being connected to the slit line.

4. The skin patch sheet according to any preceding claim, wherein in the grip section, the area of the release liner is larger than the area of the adhesive patch.

5. The skin patch sheet according to any preceding claim, wherein the grip section comprises only a carrier sheet.

6. The skin patch sheet according to any preceding claim, wherein the slit of (f) does not penetrate into the release liner.

7. The skin patch sheet according to any preceding claim, wherein the thickness of the backing is 1 to 10 µm.

8. The skin patch sheet according to any preceding claim, wherein the backing is an elastomer film, preferably a polyurethane elastomer film.

9. The skin patch sheet according to any preceding claim, wherein the carrier sheet is a polyester film, a polyolefin film or a plastic laminated paper, and the release liner is a polyester film, a polyolefin film or a plastic laminated paper, all of which are release-treated.

10. The skin patch sheet according to any preceding claim, wherein the adhesive patch has an approximately rectangular shape in which the ratio of lengths of the shorter edge and the longer edge is 1:2 to 1:9, and the slit line is formed in a direction parallel to the shorter edge at a distance in the range of 1/8 to 1/3 of the length of the longer edge of the adhesive patch; and
preferably wherein the vicinities of vertexes of the adhesive patch having an approximately rectangular shape are cut out to an approximately arc shape.

11. The skin patch sheet according to any preceding claim, wherein the carrier sheet in the grip section forms an angle of 1° to 90° with respect to the extended direction of the surface of the body section, with the slit line serving as the fulcrum.

12. The skin patch sheet according to any preceding claim, wherein the peeling force required to detach the carrier sheet from the backing is larger than the peeling force required to detach the release liner from the adhesive layer.

13. The skin patch sheet according to any preceding claim, wherein a pharmacologically active substance is included in the adhesive layer.

14. The skin patch sheet according to any preceding claim, wherein the adhesive patch is used by being attached to the face, preferably wherein the adhesive patch is used by being attached to the face for a lift-up effect; or
wherein the skin patch sheet is used to prevent direct scratching of the skin.

## Patentansprüche

1. Eine Hautpflasterfolie, die eine Trägerfolie (1), ein Klebepflaster und ein Trennpapier (4) umfasst, die in dieser Reihenfolge laminiert sind, wobei
(a) das Klebepflaster zwei Schichten aus einem Untergrund (2) und einer Klebeschicht (3) umfasst, die die gleiche Form aufweisen,
(b) die Trägerfolie an den Untergrund des Klebepflasters angrenzt,
**dadurch gekennzeichnet, dass**
(c) die Hautpflasterfolie einen Greifabschnitt (a) und einen Hauptabschnitt (b) umfasst,
(d) in dem Hauptabschnitt die Fläche des Trennpapiers größer ist als die Fläche des Klebepflasters,
(e) die Grenze zwischen dem Greifabschnitt und dem Hauptabschnitt eine Schlitzlinie ist, die von einem Schlitz (x) gebildet wird, und
(f) der Schlitz von der Trennpapierseite in die Hautpflasterfolie einschneidet, wobei er in das Klebepflaster eindringt, bis zu einer Position, an der mindestens die Hälfte oder mehr der Dicke der Trägerfolie auf dem Klebepflaster ungeschnitten bleibt.

2. Die Hautpflasterfolie gemäß Anspruch 1, wobei der Schlitz sich von der Trennpapierseite erstreckt, wobei er in das Klebepflaster eindringt, bis zu einer Tiefe im Bereich von größer als oder gleich 20% und weniger als 50% der Dicke der Trägerfolie auf der Klebepflasterseite.

3. Die Hautpflasterfolie gemäß Anspruch 1 oder Anspruch 2, wobei mehrere Folien der Klebepflaster, die jeweils eine darauf laminierte Trägerfolie aufweisen, parallel auf dem Trennpapier angeordnet sind; und wobei vorzugsweise die äußere Peripherie des Greifabschnitts gebildet wird, indem sie mit der Schlitzlinie verbunden wird.

4. Die Hautpflasterfolie gemäß einem der vorhergehenden Ansprüche, wobei in dem Greifabschnitt die Fläche des Trennpapiers größer ist als die Fläche des Klebepflasters.

5. Die Hautpflasterfolie gemäß einem der vorhergehenden Ansprüche, wobei der Greifabschnitt nur eine Trägerfolie umfasst.

6. Die Hautpflasterfolie gemäß einem der vorhergehenden Ansprüche, wobei der Schlitz von (f) nicht in das Trennpapier eindringt.

7. Die Hautpflasterfolie gemäß einem der vorhergehenden Ansprüche, wobei die Dicke des Untergrunds 1 bis 10 µm beträgt.

8. Die Hautpflasterfolie gemäß einem der vorhergehenden Ansprüche, wobei der Untergrund ein Elastomerfilm, vorzugsweise ein Polyurethanelastomerfilm ist.

9. Die Hautpflasterfolie gemäß einem der vorhergehenden Ansprüche, wobei die Trägerfolie ein Polyesterfilm, ein Polyolefinfilm oder ein laminiertes Kunststoffpapier ist und das Trennpapier ein Polyesterfilm, ein Polyolefinfilm oder ein laminiertes Kunststoffpapier ist, die alle trennbehandelt sind.

10. Die Hautpflasterfolie gemäß einem der vorhergehenden Ansprüche, wobei das Klebepflaster eine ungefähr rechteckige Form aufweist, in der das Verhältnis der Längen der kürzeren Kante und der längeren Kante 1:2 bis 1:9 beträgt und die Schlitzlinie in einer Richtung gebildet ist, die parallel zu der kürzeren Kante in einem Abstand im Bereich von 1/8 bis 1/3 der Länge der längeren Kante des Klebepflasters verläuft; und
wobei vorzugsweise die Umgebungen der Scheitel der Klebepflaster, die eine ungefähr rechteckige Form aufweisen, zu einer ungefähren Bogenform ausgeschnitten sind.

11. Die Hautpflasterfolie gemäß einem der vorhergehenden Ansprüche, wobei die Trägerfolie in dem Greifabschnitt einen Winkel von 1° bis 90° in Bezug auf die verlängerte Richtung der Oberfläche des Hauptabschnitts bildet, wobei die Schlitzlinie als der Drehpunkt dient.

12. Die Hautpflasterfolie gemäß einem der vorhergehenden Ansprüche, wobei die Abziehkraft, die zum Ablösen der Trägerfolie von dem Untergrund erforderlich ist, größer ist als die Abziehkraft, die zum Ablösen des Trennpapiers von der Klebeschicht erforderlich ist.

13. Die Hautpflasterfolie gemäß einem der vorhergehenden Ansprüche, wobei eine pharmakologisch wirksame Substanz in der Klebeschicht eingeschlossen ist.

14. Die Hautpflasterfolie gemäß einem der vorhergehenden Ansprüche, wobei das Klebepflaster verwendet wird, indem es auf dem Gesicht angebracht wird, wobei das Klebepflaster vorzugsweise verwendet wird, indem es für einen anhebenden Effekt auf dem Gesicht angebracht wird, oder
wobei die Hautpflasterfolie verwendet wird, um ein direktes Kratzen der Haut zu verhindern.

## Revendications

1. Feuille pour timbre cutané comprenant une couche vectrice (1), un timbre adhésif et une membrane de contrôle de la libération (4) laminés dans cet ordre, dans laquelle
(a) le timbre adhésif comprend un support à deux couches (2) et une couche adhésive (3) ayant la même forme,
(b) la couche vectrice est adjacente au support du timbre adhésif, **caractérisée en ce que**,
(c) la feuille pour timbre cutané comprend une section agrippement (a) et une section corps (b),
(d) dans la section corps, la surface de la membrane de contrôle de la libération est plus grande que la surface du timbre cutané,
(e) la limite entre la section agrippement et la section corps est une ligne de coupe formée par une fente (x), et
(f) la fente coupe la feuille pour timbre cutané à partir du côté de la membrane de contrôle de la libération, en pénétrant dans le timbre cutané, jusqu'à une position à laquelle au moins la moitié ou plus de l'épaisseur de la couche vectrice sur le timbre adhésif est laissée non coupée.

2. Feuille pour timbre cutané selon la revendication 1, dans laquelle la fente s'étend du côté de la membrane de contrôle de la libération, en pénétrant dans le timbre adhésif, jusqu'à une profondeur comprise entre les limites de 20 % ou plus et de moins de 50 % de l'épaisseur de la couche vectrice sur le côté adhésif du timbre.

3. Feuille pour timbre cutané selon la revendication 1 ou la revendication 2, dans laquelle multiples couches des timbres cutanés, chacune étant munie d'une couche vectrice à laquelle elle est laminée, sont disposées en parallèle sur la membrane de contrôle de la libération ; et, de préférence, dans laquelle la périphérie extérieure de la section agrippement est formée en étant reliée à la ligne de coupe.

4. Feuille pour timbre cutané selon l'une quelconque des revendications précédentes, dans laquelle, dans la section agrippement, la surface de la membrane de contrôle de la libération est plus grande que la surface du timbre adhésif.

5. Feuille pour timbre cutané selon l'une quelconque des revendications précédentes, dans laquelle la section agrippement comprend une seule couche vectrice.

6. Feuille pour timbre cutané selon l'une quelconque des revendications précédentes, dans laquelle la fente de (f) ne pénètre pas dans la membrane de contrôle de la libération.

7. Feuille pour timbre cutané selon l'une quelconque des revendications précédentes, dans laquelle l'épaisseur du support est comprise entre 1 et 10 µm.

8. Feuille pour timbre cutané selon l'une quelconque des revendications précédentes, dans laquelle le support est une pellicule en élastomère, de préférence une pellicule en élastomère de polyuréthane.

9. Feuille pour timbre cutané selon l'une quelconque des revendications précédentes, dans laquelle la couche vectrice est une pellicule en polyester, une pellicule en polyoléfine ou un papier laminé de matière plastique, et la membrane de contrôle de la libération est une pellicule en polyester, une pellicule en polyoléfine ou un papier laminé de matière plastique, toutes étant traitées pour contrôler la libération.

10. Feuille pour timbre cutané selon l'une quelconque des revendications précédentes, dans laquelle le timbre adhésif a une forme approximativement rectangulaire dans laquelle le rapport entre les longueurs du bord le plus court et le bord le plus long est de 1/2 à 1/9, et la ligne de coupe est formée dans un sens parallèle au bord le plus court à une distance comprise entre 1 huitième (1/8) et un tiers (1/3) de la longueur du bord le plus long du timbre adhésif ; et
de préférence, dans laquelle les alentours des sommets du timbre adhésif ayant une forme approximativement rectangulaire sont coupés en une forme approximativement arquée.

11. Feuille pour timbre cutané selon l'une quelconque des revendications précédentes, dans laquelle la couche vectrice située dans la section agrippement forme un angle de 1° à 90° avec le sens prolongé de la surface de la section corps, la ligne de coupe servant de point d'appui.

12. Feuille pour timbre cutané selon l'une quelconque des revendications précédentes, dans laquelle la force de pelage requise pour détacher la couche vectrice du support est plus importante que la force de pelage requise pour détacher la membrane de contrôle de la libération de la couche adhésive.

13. Feuille pour timbre cutané selon l'une quelconque des revendications précédentes, dans laquelle une substance pharmacologiquement active est incluse dans la couche adhésive.

14. Feuille pour timbre cutané selon l'une quelconque des revendications précédentes, dans laquelle le timbre adhésif est utilisé en étant fixé sur le visage, de préférence dans laquelle le timbre adhésif est utilisé en étant fixé sur le visage pour un effet de raffermissement ; ou
dans laquelle la feuille pour timbre cutané est utilisée pour empêcher les égratignures directes sur la peau.
